# EUROPEAN PATENT APPLICATION

(11) **EP 1 557 475 A1**
(43) Date of publication of application: **27.07.2005**
(21) Application number: 04256227.2
(22) Date of filing: 08.10.2004
(51) Int. Cl.: C12Q 1/68, C12N 15/10

(54) **Storage of genetic information**

(30) Priority: 22.01.2004 US 763004
(71) Applicant: Genetic Legacy, LLC, Portsmouth, New Hampshire 03801 (US)
(72) Inventor: Poulin, Paul, Yor , Maine 03909 (US)
(74) Representative: Miles, John Stephen

(57) **Abstract**

Methods for the long-term storage of hereditary information such as DNA are described herein. In some instances, lyophilized DNA and sugar is introduced into a hermetically sealed vial. Kits for the long-term storage of hereditary information are also described herein.

## Description

### BACKGROUND

This invention relates to the long-term storage of personal and hereditary information, in particular the long-term storage of DNA.

The ability to study and evaluate genetic risk factors is rapidly increasing. For example, many tests are currently available that can reveal the likelihood of suffering in later life a disease to which a person has a propensity. Some examples of these diseases include cystic fibrosis, Huntington's disease, Gaucher's disease, hemophilia, retardation, sickle cell anemia, and breast cancer.

Information from parents and grandparents is seldom collected and preserved for use in genetic testing. However, in many diseases, for example, prostatic cancers and Alzheimer's disease, it is recognized that there are multiple genes involved. Moreover, it is known that specific disease characteristics may be common for more than one disease. Accordingly, it is desirable that a multiplicity of DNA samples is available in order to identify or predict a disease in a subject. It is also desirable to have an amount of DNA sufficient to use for multiple purposes and in multiple assays.

### SUMMARY

The present invention provides methods for the long-term storage of hereditary information such as DNA. The DNA makes up the genotype of an individual (i.e., the genetic constitution), which generally correlates with the phenotype of that individual (i.e., observable characteristics such as intelligence, body make-up, propensity to certain diseases, etc.). In some instances; the invention provides for the storage of 100 µg or more of DNA, which can be used in multiple biochemical assays. Moreover, in general, the methods described herein do not require the use of a centralized storage facility, but rather allow for the storage of DNA under ambient conditions without special facilities and thus is ideal for storage at the home of a subject, or a designated individual e.g., heir of the subject.

In one aspect, the method features a method for long-term storage of hereditary information. The method includes providing a genetic sample including lyophilized DNA and sugar, the DNA being substantially free of magnesium and with the lyophilized DNA stored in a hermetically sealed UV blocking container under an inert gas.

In some instances, the sugar is a monosaccharide or a disaccharide (e.g. a disaccharide such as trelose or sucrose).

In some instances, the inert gas is nitrogen or argon.

In some instance, the UV blocking container includes borosilicate.

In some instances, the sample includes greater than 20 µg of DNA (e.g., greater than 100 µg of DNA or between about 150 and 200 µg of DNA).

In some instances, the method also includes lyophilizing the DNA.

In some instances, the DNA is obtained from the blood of a subject.

In some instances, the method also includes storing the DNA at a temperature between about -7 °C to about 24 °C.

In some instances, the sample also includes TRIS or EDTA.

In some instances, the DNA is genomic DNA.

In some instances, the method also includes isolating DNA.

In one aspect, the invention features a kit for the long-term storage of hereditary information. The kit includes a box having a first compartment having an aperture that can hold at least one hermetically sealed container, the container including lyophilized DNA of a subject and sugar; and a second portion that can hold a computer readable medium.

In some instances, the holder member is disposed in the aperture and the holder member holds 4 hermetically sealed containers of the lyophilized DNA of the subject and sugar.

In some instances the computer readable medium is an optical storage medium, for example, a DVD or CD-ROM.

In some instances, the computer readable medium is a flash memory card.

The computer readable medium can include, for example stored medical history of the subject.

In some instances, the kit is made of cardboard.

In some instances, the holder member is made of a transparent plastic.

In some instances, the hermetically sealed container is a borosilicate ampoule or borosilicate vial.

In another aspect, the invention features a method of storing hereditary information of a subject. The method includes providing a container to a party; obtaining a blood sample of a subject in the container; isolating DNA from the blood sample and lyophilizing the DNA; storing the lyophilized DNA from the blood sample in a hermetically sealed container; and providing the party with the stored, lyophilized DNA.

In some instances, the method also includes lyophilizing a solution of DNA and a sugar.

In some instances, the method also includes storing personal information of the subject on a computer readable medium.

In some instances the method also includes providing the stored personal information of the subject to the party. The personal information can include, for example, at least one of medical history, family history, personal achievements, diplomas or photographs.

In some instances, the lyophilized DNA is provided in the form of a kit.

In another aspect, the invention features a method for long-term storage of hereditary information. The method includes providing a genetic sample comprising lyophilized DNA and sugar, the DNA being substantially free of magnesium and with the lyophilized DNA stored in a hermetically sealed UV blocking container under an inert gas; and placing the sealed UV blocking container into a holding member that is inserted into an aperture of a box that together forms kit for the long-term storage of hereditary information.

In some instances the sugar is a monosaccharide or a disaccharide.

In some instances, the inert gas is nitrogen or argon.

In some instances, the sample includes greater than 20 µg of DNA.

In some instances, the method also includes storing the DNA at a temperature between about -7 °C to about 24 °C.

In some instances the DNA is genomic DNA.

In some instances the holder member holds 4 hermetically sealed containers of the lyophilized DNA and sugar.

In some instances, the method also includes disposing on the box a computer readable medium embodying personal information of the subject whose DNA is stored in the hermetically sealed containers.

In some instances the box is made of cardboard.

In some instances the holder member is made of a transparent plastic.

As used herein, the term "DNA" refers to a polynucleotide formed from covalently linked deoxyribonucleotide units. In general, DNA stores hereditary information within a cell and is the carrier of this information from generation to generation.

As used herein, the term "lyophilizing" refers to the freeze drying of a sample.

As used herein, the term "enzyme" refers to a protein that catalyzes a biochemical reaction.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a flow chart depicting a method of long-term storage of DNA.
FIG. 2 is a pictorial representation of a kit for the long term storage of DNA.
FIG. 3 is a flow chart depicting a method of obtaining a sample of DNA for long-term storage.
FIGs 4 and 5 are pictures of gels depicting the purity of samples of DNA.

### DETAILED DESCRIPTION

A method of long-term storage of DNA includes introducing lyophilized DNA and a sugar (e.g., a monosaccharide or a disaccharide) into a hermetically sealed container (e.g., a sealed vial). Moreover, in many cases, the DNA is substantially free of magnesium, a mineral involved in the enzymatic cleavage of DNA (e.g., via DNases).

The hermetically sealed container is generally a vial made from a UV blocking material such as borosilicate, coated glass, Plexiglas, or a coated metal alloy.

In general, a quantity of DNA sufficient for biological testing is stored. In some instances, for example where more DNA is required for a biological assay than is stored, the quantity of DNA can be amplified using standard PCR procedures known in the art. In general, greater than 20 µg of DNA is stored (e.g., greater than 50 µg, greater than 100 µg, greater than 150 µg or greater than 200 µg).

In order to prevent or reduce degradation of the DNA, the vial is sealed in an atmosphere of an inert gas such as nitrogen or argon. It is also generally desirable for the vial to be substantially free of moisture. Accordingly, it is desirable to maintain a hermetic seal of the vial, thus avoiding tampering with the cap of the vial or breaking the vial, which could create an opening of the vial and allow air and/or moisture to interact with the DNA sample.

The DNA sample does not require storage at low temperature (e.g., -20 °C, -70 °C, or below), but rather can be stored at room temperature. For example, the vial containing the DNA can be stored in the home of a subject. In general, the DNA sample is stored at a temperature between about -7 °C to about 24 °C. In some instances, the DNA sample is preferably stored in a cool dry place. Wide shifts in temperatures are generally avoided during the long-term storage of the DNA sample.

The method includes lyophilizing the DNA. DNA is solubilized in a solution including a buffer (e.g., a standard buffer such as TRIS or EDTA) and a sugar, such as a monosaccharide, a disaccharide or a combination thereof (e.g., about a 10% solution of disaccharide) and the DNA sample is lyophilized. For example, the DNA sample is frozen at about -60 °C in a vacuum. One of skill in the art would understand that other temperatures are suitable for the freezing of the DNA sample. For example, any temperature to maintain a frozen sample is suitable. The vacuum can be at least about 200 mTorr (e.g., about 250 mTorr or about 300 mTorr) or at most about 500 mTorr (e.g., at most about 450 mTorr, or about 400 mTorr).

Examples of sugars present in the DNA solution during lyophilization of the DNA sample include but are not limited to monosaccharides and disaccharides such as trelose or sucrose.

In some instances, the lyophilization is done in small aliquots of sample (e.g., about 0.5 ml or less) in borosilicate ampoules. The ampoules are flushed with an inert gas such as nitrogen or argon and are hermetically sealed.

In some instances the method includes isolating DNA from a sample, for example from the blood of a subject. Alternatively, the sample can be obtained from other biological samples such as a mouth swab, a tissue biopsy, muscle, bone, or skin of a subject. In general, a greater quantity of DNA can be isolated from a vial of blood (e.g., an 8 cc vial of blood) than can be isolated from a mouth swab of a subject. Accordingly, where larger quantities of DNA are required, it is preferred to isolate the DNA from a sample containing larger quantities of DNA, such as isolating the DNA from a sample of blood.

As used herein, the term isolated means to remove a sufficient number of contaminants to allow the biological manipulation and testing of the DNA, for example PCR and other biochemical or enzymatic reactions.

In some instances, for example when the DNA is isolated from the blood of a subject, the DNA is isolated and/or purified using a PAXgene Blood Validation Kit. Alternatively, the DNA is isolated using other methods known to one of skill in the art including those described by Sambrook et al., *Molecular Cloning: A Laboratory Manual,* Cold Spring Harbor, N.Y. (1989); or using Chelexe® 100 (BioRad, Richmond, Calif.), a chelating resin that has a high affinity for polyvalent metal ions.

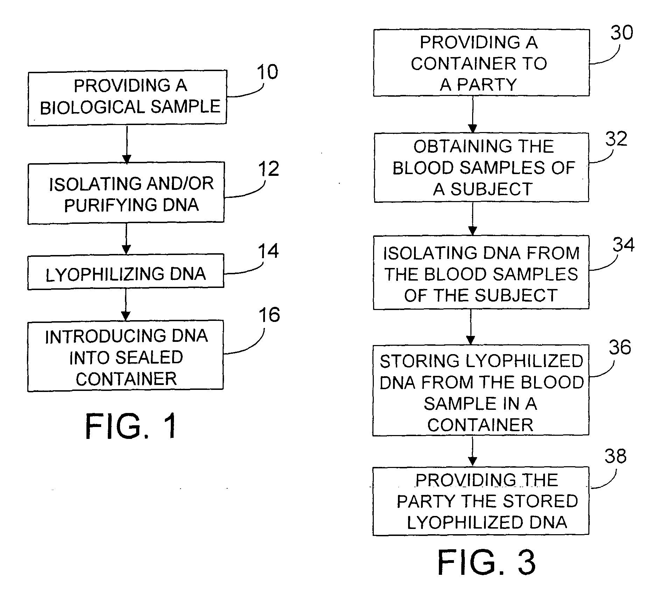

Referring to FIG. 1, an example of a method of long-term storage of DNA is shown. A biological sample from a subject is provided 12. In some instances, the subject himself provides 12 the biological sample. In other instances a biological sample from a subject is obtained from a party other than the subject. For example, in instances where the subject is deceased, a relative, trustee or heir can obtain the biological sample from a subject.

After the sample is obtained, DNA is isolated 12 from the biological sample. In some instances the DNA is purified to a desirable degree as can be measured using standard biochemical techniques such as evaluation via UV (e.g., pure DNA has an absorbance ratio at A260/A280 of between 1.7 and 1.9). In cases where the DNA is purified using the PAXgene Blood DNA Systems, the DNA is generally free of protein and other contaminants that can inhibit PCR or other enzymatic reactions.

The DNA is lyophilized 14. In general the DNA is solubilized in a buffer and sugar solution and frozen at reduced pressure. The solution is free of magnesium, which is involved in the enzymatic cleavage of DNA. For example, in some instances, the DNA is solubilized in an EDTA buffer solution containing about 10% of trelose or sucrose and frozen at a temperature of about -60 °C and pressure of 200-400 mTorr for several hours.

The lyophilized DNA sample is introduced 16 into a container and hermetically sealed. In general, the container is a UV blocking container such as a vial or ampoule and is sealed under an inert atmosphere for example using nitrogen or argon. In general, the container is substantially free of moisture.

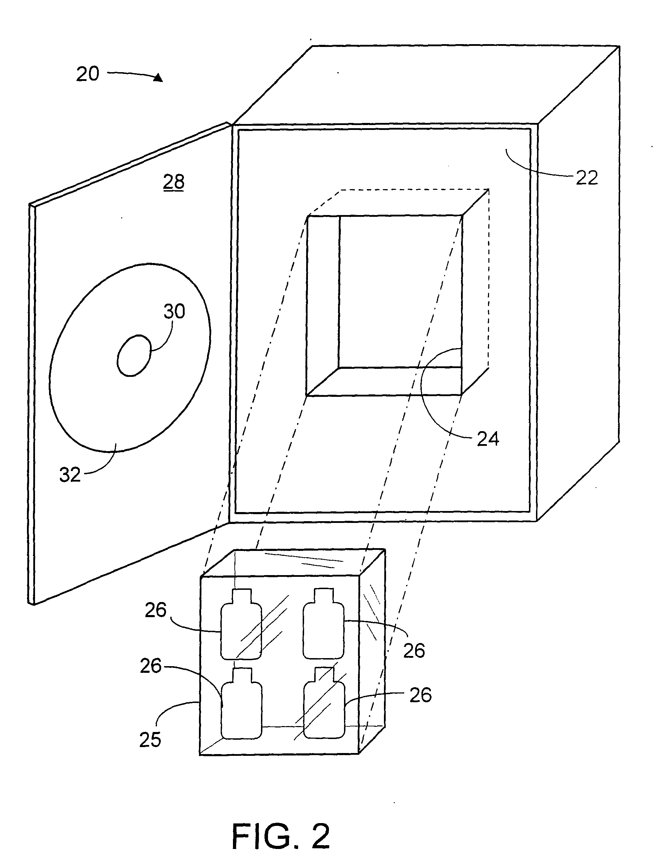

Referring to FIG. 2, a kit 20 for the long-term storage of hereditary information is shown. The kit 20 is in the general form of a box and with a hinged cover. The kit includes a first compartment 22 that includes an aperture 24. The aperture 24 received a holder member 25 that holds one or more containers 26 such as vials that include lyophilized DNA, e.g., of the subject of the kit. As described above, the containers 26 are generally hermetically sealed and are made from a UV blocking material such as borosilicate.

The kit 20 also includes a second portion 28 that can hold a computer readable medium such as a flash memory device or an optical storage medium. As shown in FIG. 2, the second portion 28 can be a hinged member that includes a protrusion 30 that secures optical storage medium 32 such as a DVD or a CD-ROM. Alternatively, the second portion 28 can be a sleeve that fits inside the first compartment 22. In some instances, the computer readable medium is physically attached to the second portion 28, and in other instances the computer readable medium is positioned within the sleeve of the second portion 28.

The first compartment 22 can be made from a material such as cardboard or plastic. In general, the second portion 28 can be made from the same material as the first compartment 22. Alternatively, the second portion 28 is made from a material different than that of the first compartment 22. The holder member 25 can be made from a transparent material, e.g., a plastic and can have recesses molded therein in to securely hold the vials.

In some instances the holder member 25 holds 1, 2, 3, 4, or more containers that include lyophilized DNA.

Personal information about the subject can be included in the kit and stored on an electronic storage medium such as a flash memory chip or an optical storage medium including a DVD or a CD-ROM. Examples of personal information include but are not limited to a biography, an obituary, a family tree, medical history, copies of photographs such as family photographs and other documents such as diplomas, certificates and/or honors.

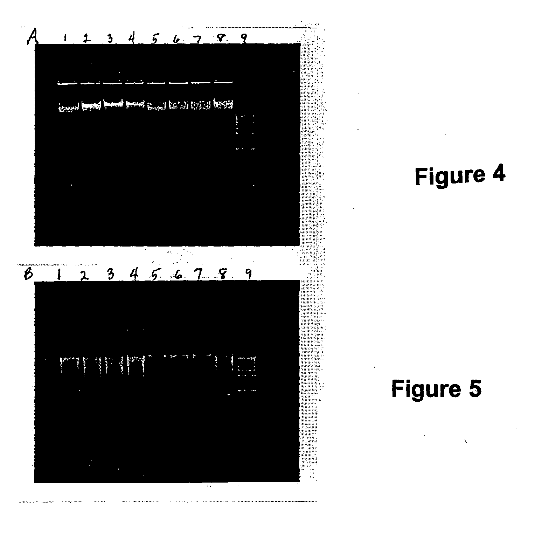

Referring to FIG. 3 a method of obtaining a sample of DNA and producing a kit is shown. A party is provided 30 a container. For example, a party can contact a person or organization that provides services that include the processing and or storing of DNA. The person or organization provides 32 to the party a container that can hold a sample of blood. For example, the container can be sized and shaped to hold an 8cc sample of blood such as is typically obtained in a standard blood draw. In some instances, the person or organization provides the container together with packaging that will enable the safe mailing of the blood sample, for example, to a biological processing site. As radiating the blood sample can damage the DNA, when mailing the sample, it is desirable to use a shipping service that does not irradiate the package.

The DNA is isolated and/or purified 34 from the blood sample as discussed above. DNA can be isolated from a blood sample using any technique known to one of skill in the art. In some instances the DNA is isolated using a commercially available kit such as PAXgene Blood DNA Systems.

The DNA is lyophilized and stored 36 in a hermetically sealed container using the methods for lyophilization and storage as described above. In general, the DNA is lyophilized in a sugar solution such as a 10% sucrose solution where the solution is substantially free of magnesium.

The stored DNA sample is provided 38 to the requesting party in the hermetically sealed container under conditions that do not require special storage treatments or equipment but instead can be stored in the home of the party. However, it is generally preferred that the DNA be stored in a cool and dry place.

In some instances the party sends to the person or organization personal information about the subject whose DNA is being stored. In some instances, the stored sample of DNA is the DNA of the requesting party. In other instances, the stored DNA is the DNA of a third party such as a friend, relative, patient, etc.

The personal information can be sent to the person or organization through mail, fax, electronic or other forms. Examples of personal information and personal history include documents such as birth certificates, obituaries, diplomas, etc. Other person information includes pictures of the subject and/or pictures of the subject's family. Medical information, including medical history, can also be sent to the person or organization. Information sent to the person or organization is stored on a computer readable medium such as a flash memory card or an optical storage medium (e.g., a DVD or CD-ROM) and provided to the party. The organization can include a web site that through a web browser can conduct an interview with the requesting party to obtain information about the subject. The personal information and personal history embodied as documents can be sent to the organization view the web browser interview, e.g., by scanning the documents and attaching them to an E-mail that is directed to an address given during the interview.

In some instances the DNA is provided to the party in the form of a kit, for example a kit described herein. In instances where personal information has been provided, the personal information in the form of a computer readable medium is also included in the kit.

### Materials and Methods

### Isolation of Genomic DNA from blood

Venous blood is collected in an 8.5 ml PAXgene blood collection tube and provided to a biological facility. The sample was not stored for longer than 14 days at 15-25 °C. Where the blood was stored short term (up to about four weeks) it was stored at 2-8 °C. In instances where the blood was stored longer than four weeks, the blood was frozen, for example at -20 °C or -70 °C.

1.4 ml of Buffer BG4 resuspension buffer is added with a sterile pipette to lyophilized preAnalytix protease. For each sample, 5 mls of Buffer BG3 digestion buffer was reconstituted with 50 µl of preAnalytix protease and prepared immediately prior to the processing of the blood sample.

The blood sample is poured into a 50 ml processing tube containing the BG1 lysis buffer and the mixture mixed by inverting the tube about 5 times. The mixture is then centrifuged for 5 minutes at 2500xg in a swing-out rotor. The supernatant is carefully discarded while not loosing the button of sample at the bottom of the tube.

5 ml of Buffer BG2 was added with a sterile pipette and the tube was closed. The button was washed by vortexing vigorously for 5 seconds. The sample was then centrifuged for 3 minutes at 2500xg in a swing-out rotor and the supernatant was carefully discarded.

5 ml of Buffer BG3/PreAnalytix Protease was added and the tube was closed and vortexed on high for 20 seconds or for a time sufficient to dissolve the button completely. The tube was then placed in a heating block or water bath and incubated at 65 °C for ten minutes. The sample changed color from light red to light green, indicating that protein digestion had occurred. The sample was then vortexed again at high speed for five seconds.

5 ml of isopropanol (100%) was added and mixed with the sample by inversion of the tube about 20 times or until the white DNA strands clumped visibly together. The sample was centrifuged for 3 minutes at 2500xg and the supernatant discarded. The tube was left inverted on a clean piece of absorbent paper for 1 minute.

5 ml of 70% ethanol was added and vortexed for 1 second at high speed and the sample was subsequently centrifuged for 3 minutes at 2500xg. The supernatant was discarded and the tube left inverted on a clean piece of absorbent paper for at least 5 minutes. The tube was dabbed onto absorbent paper to remove ethanol from the rim and left inverted for another 5 minutes to allow the DNA button to dry.

1 ml of Buffer BG4 was added and the DNA dissolved by incubating 1 hour at 65 °C in a heating block or water bath, followed by incubation at room temperature overnight.

### Evaluation of Recovered DNA

DNA yield is determined from the concentration of DNA in the eluant, measured by absorbance at 260 nm. Absorbance readings at 260 nm, in order to be accurate, fall between 0.1 and 1.0. BG4 was used as appropriate to dilute samples and to calibrate the spectrophotometer. The absorbance was measured at 260nm and 280nm, or in some instances the absorbance was scanned from 220 to 320 nm.

DNA purity was determined by calculating the ratio of absorbance at 260 nm to absorbance at 280 nm. Pure DNA has an A260 / A 280 ratio of 1.7 to 1.9. DNA purified by the PAXgene blood DNA systems procedure is generally free of protein and other contaminants that can inhibit PCR or other enzymatic reaction. The purified DNA can be used immediately or stored in Buffer BG4 at -20 °C for later use.

### Determination of DNA length:

The length of the purified DNA can be determined by comparison with DNA markers of known size, for example, using pulsed-field agarose gel electrophoresis (PFGE) and ethidium bromide staining. 1-5 micrograms per well is loaded. Standard PFGE are as follows: 1% agarose gel in a 0.5x TBE electrophoresis buffer (45 mM Tris-borate; 1mM EDTA, pH 8.0); switch intervals, 5-40 seconds; run time 12 hours; voltage, 170V. The expected size of genomic DNA purified using the PAX gene Blood DNA system is up to 200 kb (predominately 50-150 kb).

### EXAMPLES

### DNA Results: Amounts, Purity, Sensitivity to Restriction Enzyme Digestion

The following subjects' DNA was obtained using the methods described above. The yield and purity are provided in Table 1 below.

**Table 1:**

| DNA Yield and Purity | | |
|---|---|---|
| SUBJECT | YIELD (micrograms) | OD RATIO 260/280 nm |
| A | 166 | 1.95 |
| B | 217 | 1.92 |
| C | 184 | 1.89 |
| D | 374 | 1.89 |

Samples of each DNA preparation were digested with the restriction enzyme EcoRi as a measure of purity of the DNA and depicted in Figures 3 and 4. Sensitivity of the DNA to enzyme digestion, reflected in its fragmentation to a smear from a single large band, indicates that the DNA is clean enough (i.e., pure enough) for use in a variety of techniques. Analysis by gel electrophoresis can also reveal the presence of contaminating RNA.

A number of embodiments of the invention have been described. Nevertheless, it will be understood that various modifications may be made without departing from the spirit and scope of the invention. Accordingly, other embodiments are within the scope of the following claims.

## Claims

1. A method for long-term storage of hereditary information comprising,
providing a genetic sample comprising lyophilized DNA and sugar, the DNA being substantially free of magnesium and with the lyophilized DNA stored in a hermetically sealed UV blocking container under an inert gas.

2. The method of claim 1 wherein the sugar is a monosaccharide or a disaccharide.

3. The method of claim 2 wherein the sugar is a disaccharide.

4. The method of claim 3 wherein the disaccharide is trelose or sucrose.

5. The method of claim 1 wherein the inert gas is nitrogen or argon.

6. The method of claim 1 wherein the UV blocking container comprises borosilicate.

7. The method of claim 1 wherein the sample comprises greater than 20 µg of DNA.

8. The method of claim 1 wherein the sample comprises greater than 100 µg of DNA.

9. The method of claim 1 wherein the sample comprises. between about 150 and 200 µg of DNA.

10. The method of claim 1 further comprising lyophilizing the DNA.

11. The method of claim 1 wherein the DNA is obtained from the blood of a subject.

12. The method of claim 1 further comprising storing the DNA at a temperature between about -7 °C to about 24 °C.

13. The method of claim 1 wherein the sample further comprises TRIS or EDTA.

14. The method of claim 1 wherein the DNA is genomic DNA.

15. The method of claim 1 further comprising isolating DNA.

16. A kit for the long-term storage of hereditary information comprising
a box having a first compartment having an aperture that can hold at least one hermetically sealed container, the container comprising lyophilized DNA of a subject and sugar;. and a second portion that can hold a computer readable medium.

17. The kit of claim 16 wherein a holder member is disposed in the aperture and the holder member holds 4 hermetically sealed containers of the lyophilized DNA of the subject and sugar.

18. The kit of claim 16 wherein the computer readable medium is an optical storage medium.

19. The kit of claim 18 wherein the optical storage medium is a DVD or a CD-ROM.

20. The kit of claim 16 wherein the computer readable medium is a flash memory card.

21. The kit of claim 16 wherein the computer readable medium includes stored medical history of the subject.

22. The kit of claim 16 wherein the kit is comprised of cardboard.

23. The kit of claim 16 wherein the holder member is comprised of a transparent plastic.

24. The kit of claim 16 wherein the hermetically sealed container is a borosilicate ampoule or borosilicate vial.

25. A method of storing hereditary information of a subject comprising:
providing a container to a party;
obtaining a blood sample of a subject in the container;
isolating DNA from the blood sample and lyophilizing the DNA;
storing the lyophilized DNA from the blood sample in a hermetically sealed container; and
providing the party with the stored, lyophilized DNA.

26. The method of claim 25 further comprising lyophilizing a solution of DNA and a sugar.

27. The method of claim 25 further comprising storing personal information of the subject on a computer readable medium.

28. The method of claim 27 further comprising providing the stored personal information of the subject to the party.

29. The method of claim 27 wherein the personal information includes at least one of medical history, family history, personal achievements, diplomas or photographs.

30. The method of claim 27 wherein the lyophilized DNA is provided in the form of a kit.

31. A method according to any of claims 1, 2, 5, 7, 12 or 14, further comprising
placing the sealed UV blocking container into a holding member that is inserted into an aperture of a box that together forms kit for the long-term storage of hereditary information.

32. The method of claim 31 wherein the holder member holds 4 hermetically sealed containers of the lyophilized DNA and sugar.

33. The method of claim 16 further comprising:
disposing on the box a computer readable medium embodying personal information of the subject whose DNA is stored in the hermetically sealed containers.

34. The method of claim 31 wherein the box is comprised of cardboard.

35. The method of claim 31 wherein the holder member is comprised of a transparent plastic.
